(19) Europäisches Patentamt
European Patent Office
Office européen des brevets

(11) Publication number: **0 367 364 B1**

(12) **EUROPEAN PATENT SPECIFICATION**

(45) Date of publication of patent specification: **02.02.94**

(51) Int. Cl.5: **C07C 255/25**, C07C 253/00

(21) Application number: **89250068.7**

(22) Date of filing: **31.10.89**

(54) **Integrated process for the production of aminoacetonitriles.**

(30) Priority: **31.10.88 US 264413**

(43) Date of publication of application:
**09.05.90 Bulletin 90/19**

(45) Publication of the grant of the patent:
**02.02.94 Bulletin 94/05**

(84) Designated Contracting States:
**AT BE CH DE ES FR GB GR IT LI LU NL SE**

(56) References cited:
**EP-A- 0 102 343**
**EP-A- 0 102 935**
**EP-A- 0 184 694**
**DE-C- 669 808**

(73) Proprietor: **HAMPSHIRE CHEMICAL CORPORATION**
**55 Hayden Avenue**
**Lexington, Massachusetts 02173(US)**

(72) Inventor: **Su, Jow-Lih**
**316 Hammonton Place**
**Silver Spring Maryland 20904(US)**
Inventor: **Sherwin, Martin Barry**
**11500 Karen Drive**
**Potomac Maryland 20854(US)**

(74) Representative: **UEXKÜLL & STOLBERG Patentanwälte**
**Beselerstrasse 4**
**D-22607 Hamburg (DE)**

EP 0 367 364 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the production of aminoacetonitriles, and more specifically to an integrated process wherein a crude, unpurified hydrogen cyanide product gas stream from a hydrogen cyanide process reactor together with a crude, unpurified formaldehyde product gas stream from a formaldehyde process reactor, are fed directly into a reactive absorber, together with an additional nitrogen source and scrubbed with a controlled-pH, aqueous solution to produce aminoacetonitriles in high yields. This process provides improved economics for the production of aminoacetonitriles by eliminating the costly recovery and purification processes associated with conventional hydrogen cyanide and formaldehyde production processes.

### Background

It is known in the prior art that aminoacetonitriles can be prepared by reacting formaldehyde and hydrogen cyanide together with a nitrogen source in liquid phase. For example, nitriloacetonitrile can be produced by this method as shown in the following general reaction:

$$NH_3 + 3\ CH_2O + 3\ HCN \xrightarrow{pH \leq 1} N(CH_2CN)_3$$

On an industrial scale, the conventional processes for the production of aminoacetonitriles require purified, commercial grade liquid hydrogen cyanide and formaldehyde in high concentration in order to obtain product in high enough yield to warrant economic feasibility. Substantial engineering considerations and capital equipment costs can be attributed to the recovery and purification equipment required to obtain commercially pure formaldehyde and hydrogen cyanide. For example, in the production of hydrogen cyanide, the crude product gas stream contains, in addition to hydrogen cyanide, a significant amount of ammonia. In conventional manufacturing methods the ammonia must always be removed to avoid the dangerous exothermic polymerization of the liquid hydrogen cyanide.

Similarly, in the production of formaldehyde, the crude reactor product stream is a dilute gaseous mixture of formaldehyde and water which requires large absorption columns to recover formaldehyde in sufficient purity.

From DE-G-669 808 it is known a process for the production of reaction products of hydrocyanic acid in which a crude stream of hydrocyanic acid is produced by pyrolysis of formamide, the gas stream is cooled and is reacted with an aqueous solution of chemical sorbents such as carbonyl compounds.

It has now been discovered that aminoacetonitriles can be produced using crude, unpurified gaseous product streams from formaldehyde and hydrogen cyanide reactors directly without the need of purifying these reactive product streams. Under the process of this invention, it is no longer necessary, and is actually redundant to remove the unreacted ammonia gas from the hydrogen cyanide product gas stream as a purification step, only to add it later in the downstream production of aminoacetonitriles. This also applies to the excess water in each of the hydrogen cyanide and formaldehyde processes. That is, since the reactants in the aminoacetonitrile production process must be diluted in water prior to their reactions, it is redundant to remove water from the crude unpurified reactor product streams.

### Summary of the Invention

An object of this invention is to provide a new and improved process for the production of aminoacetonitriles.

A further object of this invention is to provide a simple integrated process for the production of aminoacetonitriles.

A further object of this invention is to provide an improved process for the production of aminoacetonitriles wherein conventional upstream reactant purification processes are eliminated.

Under the process of this invention, it has been discovered that aminoacetonitriles can be produced by an integrated process wherein the direct, unpurified hydrogen cyanide product gas stream from a hydrogen cyanide process reactor comprising a mixture of hydrogen cyanide and ammonia and a crude, unpurified product gas stream from a formaldehyde process reactor, together with an additional nitrogen source, are

contacted under reactive conditions to produce aminoacetonitrile in high yields.

These and other objects will be apparent from the remaining specification and the appended claims.

## Detailed Description

The subject process is directed to a means of producing aminoacetonitriles by integrating the gaseous product streams from the production processes of hydrogen cyanide and formaldehyde into a simple one-step aminoacetonitriles formation process, thereby eliminating conventional formaldehyde and hydrogen cyanide purification and recovery processes.

Specifically, under the process of this invention, two reactant streams, one containing an unpurified, crude hydrogen cyanide product stream comprising a gaseous mixture of hydrogen cyanide and unreacted ammonia, the other containing a crude, unpurified gas stream from a formaldehyde process reactor comprising a gaseous mixture of formaldehyde, water and unreacted methanol, together with an additional nitrogen source, are scrubbed in a reactive absorber with an aqueous, controlled pH solution to produce aminoacetonitriles. Under this process, reactant recovery as well as reaction are performed in the same reactive absorber. The two reactant streams may be introduced into the reactive absorber through separate feed streams, or optionally, the reactant streams may be mixed together prior to being fed into the reactive absorber. When those reactant streams are pre-mixed, it is believed that glycolonitrile is formed as an intermediate. Suitable types of reactive absorbers include, but are not limited to, bubble column, packed column, tray column and the like, and is preferably a bubble column. Furthermore, for flexibility of engineering design, an optional additional reactor may be added downstream from the reactive absorber, in order to accelerate the nitrile formation reaction that was initiated in the reactive absorber. This reactor may be in the form of a stir tank or a hot tube and is preferably a hot tube. The hot tube is typically maintained at a temperature in the range 50 to 200 °C and a reactant residence time in the range 10 seconds to 1 hour. Various other reactor types are well known to those skilled in the art, and a choice of a particular reactor is not critical, per se, to this invention.

Those processes capable of forming reactant hydrogen cyanide streams suitable for use in this invention include: the ammoxidation of methane (Andrussow Process or the Degussa process, also called the BMA process), the reaction of ammonia and propane (Fluohmic process), the ammoxidation of methanol, the decomposition of formamide, and the recovery of hydrogen cyanide as the by-product in the preparation of acrylonitrile by the ammoxidation of propylene (SOHIO process). These and other similar processes are well documented in the art. Since all of these processes use ammonia as the source of nitrogen, the product gas streams will contain a mixture of unreacted ammonia and hydrogen cyanide. Contemplated equivalents are those processes that are simple variations of the given examples but produce a crude unpurified hydrogen cyanide product stream containing an excess of unreacted ammonia or related nitrogen source.

A suitable process for the production of a crude, unpurified formaldehyde reactant stream is by the catalytic dehydrogenation of methanol over a silver catalyst (BASF process). The reaction is endothermic, and may be written as follows:

$$CH_3OH \rightleftarrows CH_2O + H_2$$

Another suitable process to prepare a crude formaldehyde reactant stream is by the oxidation of methanol by a metal oxide catalyst such as ferric molybdate. This reaction using a ferric molybdate catalyst is exothermic, and may be written as follows:

$$CH_3OH + \tfrac{1}{2} O_2 \rightarrow CH_2O + H_2O$$

These crude formaldehyde product streams are introduced directly into the reactive absorber.

The additional sources of nitrogen capable of being used in this process can be represented by the formula

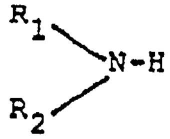

wherein $R_1$ and $R_2$ are each independently selected from hydrogen, alkyl, alkene, or cycloalkyl group, preferably a $C_1$ to $C_{20}$ alkyl or alkene and most preferably a $C_1$ to $C_3$ alkyl or alkene group. Examples of suitable nitrogen sources include, but are not limited to ammonia, methylamine, ethylamine, n-propylamine, isopropylamine, n-butylamine, isobutylamine, sec.-butylamine, tert.-butylamine, pentylamine, pentyl-2-amine, pentyl-3-amine, n-hexylamine, n-heptylamine, n-octylamine, n-nonylamine, n-decylamine and 2-ethylhexylamine; ethylenediamine, propylenediamine, isopropylenediamine, butylenediamine, sec.-butylenediamine, isobutylenediamine and tert.-butylenediamine; diethylenetriamine, dipropylenetriamine, diisopropylenetriamine, dibutylenetriamine, diisobutylene-triamine, di-sec.-butylenetriamine and ditert.-butylene-triamine; triethylenetetramine, tripropylenetetramine, triisopropylenetetramine, tributylenetetramine, tri-sec.-butylenetetramine, triisobutylenetetramine and tri-tert.-butylenetetramine, tetraethylenepentamine, tetra-propylenepentamine, tetraisopropylenepentamine, tetrabutylenepentamine, tetraisobutylenepentamine, tetra-sec.-butylenepentamine and tetra-tert.-butylenepentamine.

A first embodiment of this invention is directed to a process for the preparation of nitrilotriacetonitrile. In this embodiment a crude, unpurified hydrogen cyanide product stream and a crude unpurified formaldehyde product stream, are produced in respective upstream reactors by one of the previously disclosed processes and are fed into a reactive absorber together with an additional gaseous or liquid ammonia or hexamethylenetetramine stream (HMTA) and scrubbed with an acidified aqueous solution. The reactive absorber is maintained at a temperature between 25 and 90 °C, and preferably between 60 and 85 °C. The pressure of the reactive absorber is maximized to enhance absorption and is typically maintained in the range 0.35 to 14.1 kg/cm$^2$ (5 to 200 psig). The scrubbing solution is an acidified aqueous solution, and may contain recycled nitrilotriacetonitrile. Any acid can be used in the scrubbing solution to control the pH and is typically sulfuric acid, The acidified aqueous scrubbing solution is maintained at a pH in the range 0.1 to 2.0, and preferably in the range 0.1 to 0.5, by the continuous addition of acid.

A second embodiment of this invention is directed to a process for the preparation of ethylenediaminetetraacetonitrile (EDTN). In this embodiment a crude, unpurified hydrogen cyanide product stream and a crude, unpurified formaldehyde product stream are produced in respective upstream reactors. The product streams are fed directly into a reactive absorber together with a gaseous or liquid ethylenediamine stream and scrubbed with an acidified aqueous solution, The reactive absorber is maintained at a temperature in the range 50° to 90 °C and is preferably between 60° to 85 °C. The pressure of the reactive absorber is maximized to enhance absorption and is typically in the range 0.35 to 14.1 kg/cm$^2$ (5 to 200 psig). The acidified aqueous scrubbing solution is maintained, by the continuous addition of acid, at a pH in the range 0.1 to 2.0 and preferably 0.5 to 1.0, and may also contain some recycled EDTN.

A third embodiment of this invention is directed to a process for the preparation of glycinonitrile. In this embodiment a crude, unpurified hydrogen cyanide product stream and a crude, unpurified formaldehyde product stream are produced in respective upstream reactors. The product streams are fed directly into a reactive absorber together with an additional gaseous or liquid ammonia stream and scrubbed with an alkaline aqueous solution at a temperature in the range 25° to 85 °C. The pressure of the reactive absorber is maximized to enhance absorption and is typically maintained in the range 0.07 to 0.70 kg/cm$^2$ (1 to 10 psig). The scrubbing solution is an aqueous alkaline solution maintained at a pH in the range 8 to 12, preferably in the range 9 to 11, by the continuous addition of ammonia. The scrubbing solution may also contain some recycled glycinonitriles. The reaction mixture, which may contain glycolonitrile may optionally be pumped through a hot tube at a temperature in the range 60° to 100 °C to accelerate the production of glycinonitrile.

A fourth embodiment of this invention is directed to a process for the preparation of iminodiacetonitrile (IDAN). In this embodiment a crude, unpurified hydrogen cyanide product stream and a crude, unpurified formaldehyde product stream are produced in respective upstream reactors. The product streams are fed directly into a reactive absorber together with an additional gaseous or liquid ammonia stream and scrubbed with an acidified aqueous solution, The reactive absorber is maintained at a temperature in the range ambient to 150 °C, preferably between 25° to 90 °C and is more preferably between 50° to 70 °C. The pressure of the reactive absorber is maximized to enhance absorption and is typically in the range 0.35 to 0.70 kg/cm$^2$ (5 to 10 psig). The acidified aqueous scrubbing solution is maintained, by the continuous addition of acid, at a pH in the range 1 to 10 and preferably 6 to 8, and may also contain some recycled IDAN.

The following examples are provided to illustrate the invention in accordance with the principles of this invention, but are not to be construed as limiting the invention in any way except as indicated in the appended claims. All parts and percentages are by moles unless otherwise indicated.

Example 1

This example describes the preparation of nitrilotriacetonitrile in accordance with the process of this invention. Hydrogen cyanide and formaldehyde streams were generated in upstream reactors by methanol ammoxidation and methanol oxidation respectively. The crude, unpurified gaseous hydrogen cyanide product stream contained approximately 3-5% hydrogen cyanide with a total flow rate of approximately 10-11 moles per hour. The crude, gaseous formaldehyde stream contained approximately 4-5% formaldehyde with a total flow rate of approximately 10-11 moles per hour. These crude gaseous product streams were fed directly into a bubble column reactive absorber containing a scrubber solution containing 1500g of water, acidified to a pH in the range 0.1 to 0.5 with about 200g of sulfuric acid. An additional nitrogen source was also fed to the scrubber e.g, ammonia or HMTA, for the nitrilotriacetonitrile formation. The amount of hydrogen cyanide and formaldehyde recovered in the scrubber varied depending on the scrubber conditions. Typically the hydrogen cyanide recovery was between 61-85% and the formaldehyde recovery was greater than 98%. The reactive absorber solution was recycled with continuous addition of sulfuric acid to maintain a constant pH of about 0,1 to 0.5, and to remove excess water. The reactive absorber was maintained at a temperature of 60° to 85°C and at a pressure 0.70 to 1.41 kg/cm$^2$ (of 10 to 20 psig). Every five hours the scrubbing solution was completely withdrawn, cooled, and filtered to remove the solid nitriles. The filtrate was then charged back into the scrubber for further reaction. This experiment was run for approximately 15 to 20 hours.

| Experimental Conditions and Results | | | | | | | | | |
|---|---|---|---|---|---|---|---|---|---|
| Experiment No. | pH | Temp. °C | Pressure kg/cm$^2$ (psig) | Total reactants recov'd | | | | Total NTAN formed (Moles) | Yield Based on HCN (%) |
| | | | | $CH_2O$ | HCN | $NH_3$ | HMTA | | |
| 1 | 0.1 | 60-70 | 0.70 (10) | 4.9 | 3.3 | 1.0 | - | 0.29 | 26 |
| 2 | 0.1 | 60-70 | 0.70 (10) | 4.8 | 4.9 | 1.8 | - | 0.42 | 26 |
| 3 | 0.5 | 60-70 | 0.70 (10) | 8.6 | 7.6 | 2.3 | - | 0.56 | 28 |
| 4 | 0.1-0.3 | 85 | 1.41 (20) | 8.5 | 7.0 | 2.4 | - | 0.39 | 17 |
| 5 | 0.1-0.3 | 70 | 1.41 (20) | 4.5 | 5.4 | - | 0.45 | 0.91 | 51 |

Example 2

This example describes the preparation of ethylenediaminetetraacetonitrile in accordance with the process of this invention. Hydrogen cyanide and formaldehyde streams were generated in upstream reactors by methanol ammoxidation and methanol oxidation respectively as described in Example 1. The crude, unpurified, product gas streams from these reactors were fed directly into a bubble column reactive absorber containing 1500g of water acidified to a pH of 0.8, together with an additional stream of ethylenediamine (EDA). The reactive absorber solution was recycled with continuous addition of sulfuric acid to maintain a constant pH of about 0.8. The reactive absorber was maintained at a temperature of 60°C and at a pressure of 0.70 kg/cm$^2$ (10 psig). The results are summarized as follows:

| Total Recovered Reactants in Reactive Absorber (moles) | | | Total EDTN formed (moles) | EDTN Yield (%) based on: | | |
|---|---|---|---|---|---|---|
| EDA | $CH_2O$ | HCN | | EDA | $CH_2O$ | HCN |
| 1.0 | 3.9 | 3.8 | 0.24 | 24 | 25 | 25 |

Example 3

This example describes the preparation of glycinonitrile in accordance with the process of this invention. In this example, a separate absorption/reaction scheme was used. Hydrogen cyanide and formaldehyde product streams were generated in upstream reactors by methanol ammoxidation and

methanol oxidation respectively as described in Example 1. The crude, unpurified gaseous product streams from these reactors were fed directly into a bubble column, together with an additional stream of ammonia and scrubbed with an ammonia-water solution. The reactive absorber solution was recycled with the continuous addition of ammonia to maintain a constant pH of about 10.0 to about 10.7. The reactive absorber was maintained at a temperature of 25 to 30 ° C and a pressure of 0.28 kg/cm$^2$ (4 psig). Hydrogen cyanide was completely recovered in a 1.2192 m (4 ft). column. This suggested that the absorption was enhanced by chemical reaction, most likely by the formation of glycolonitrile.

In the ensuing reaction step, the reaction mixture from the scrubber was pumped through a hot tube at 80 ° C for 5 to 10 minutes where glycinonitrile was produced. The results are summarized as follows:

**Table 3**
**Production of Glycinonitrile**

| | | | | |
|---|---|---|---|---|
| Absorption Step | Scrubbing Liquid | 1500g $H_2O$ | 1500g $H_2O$ | 1500g $H_2O$ |
| | Gas Feed Compositing | HCN = 1.6 mol% | HCN = 1.6 mol% | HCN = 1.6 mol% |
| | | $CH_2O$ = 1.7 mol% | $CH_2O$ = 1.7 mol% | $CH_2O$ = 1.7 mol% |
| | | $NH_3$ = 4.6 mol% | $NH_3$ = 6.8 mol% | $NH_3$ = 8.9 mol% |
| | Liquid/Vapor Molar Ratio | 1.6 | 1.6 | 1.6 |
| | Temperature, °C | 27 | 27 | 27 |
| | Pressure, $kg/cm^2$ (psig) | 0.28 (4) | 0.28 (4) | 0.28 (4) |
| Reaction Step | Temperature, °C | 80 | 80 | 80 |
| | Res. Time, min. | 6.2 | 6.2 | 6.2 |
| | pH | 10.2 | 10.5 | 10.7 |
| | $NH_3$/HCN Molar Ratio | 2.9 | 4.2 | 5.6 |
| Glycinonitrile Yield, % | Based on HCN | 84.0 | 87.5 | 82.8 |
| | Based on $CH_2O$ | 79.4 | 82.4 | 77.9 |

Example 4

Hydrogen cyanide and formaldehyde were produced in respective upstream reactors, cooled and fed directly into a reactive absorber together with an additional ammonia stream and $H_2SO_4$ acidified water. The reactive absorber solution was recycled with continuous addition of $H_2SO_4$ to maintain a constant pH range

of 6.0 to 8.0. The reactive absorber was maintained at 65-70 °C, with a pressure of 0.70 kg/cm$^2$ (10 psig).

| | |
|---|---|
| Reactive Absorber Starting Solution: | 1500 g. $H_2O$ (pH 6.5) |
| Gaseous Feed Composition: | HCN: 0.321 moles/hr.<br>$CH_2O$: 0.330 moles/hr.<br>$NH_3$: 0.457 moles/hr.<br>$O_2$: 1.547 moles/hr.<br>$N_2$: 19.260 moles/hr.<br>CO: 0.287 moles/hr.<br>$H_2O$: 0.900 moles/hr. |
| Temperature:<br>Pressure:<br>pH - | 65-70 °C<br>0.70 kg/cm$^2$ (10 psig).<br>maintained at 6-8 by continuous addition of $H_2SO_4$. |

| Reaction time (Hr.) | Total IDAN formed (moles) | | IDAN Yield percent based on the absorbed: | |
|---|---|---|---|---|
| | In Solution | As Precipitate | HCN | $CH_2O$ |
| 1 | 0 | 0 | 0 | 0 |
| 2 | 0.019 | 0 | 6.0 | 5.8 |
| 3 | 0.084 | 0 | 17.4 | 16.9 |
| 4 | 0.155 | 0 | 24.2 | 23.5 |
| 5 | 0.630 | 0 | 74.7 | 72.6 |
| 8 | 0.822 | 0.211 | 80.4 | 78.1 |
| 10 | 0.787 | 0.558 | 83.8 | 81.4 |

## Example 5

The experiment as described in Example 1 was repeated except that the temperature of the reactive absorber was maintained at 60 °C.

| | |
|---|---|
| Reactive Absorber Starting Solution: | 1500 g. $H_2O$ (pH 6.5) |
| Gaseous Feed Composition: | HCN: 0.47 moles/hr.<br>$CH_2O$: 0.50 moles/hr.<br>$NH_3$ 0.30 moles/hr.<br>$O_2$ 1.34 moles/hr.<br>$N_2$ 19.76 moles/hr.<br>CO 0.27 moles/hr.<br>$H_2O$ 2.44 moles/hr. |
| Temperature:<br>Pressure:<br>pH - | 60 °C<br>0.70 kg/cm$^2$ (10 psig).<br>maintained at 6-8 by continuous addition of $H_2SO_4$. |

HCN absorption efficiency in the reactive absorber varied from 83-95%.

| Reaction Time (Hr.) | IDAN Formed (Moles) | IDAN Yield Percent (based on absorbed hydrogen cyanide) |
|---|---|---|
| 0 - 5.5 | 0.96 [(a)] | 86 % |
| 5.5-11.5 | 0.95 [(b)] | 79 % |
| 11.5-16.5 | 0.72 [(b)] | 62 % |
| 16.5-21.5 | 0.85 [(b)] | 85 % |

(a) dissolved in scrubbing solution
(b) recovered as precipitate

## Claims

1. A process for the production of aminoacetonitriles prepared by
   a) contacting in a reactive absorber, under reactive conditions hydrogen cyanide, formaldehyde, and an additional nitrogen source,
   b) scrubbing the reaction products from (a) supra with a pH-controlled, aqueous scrubbing solution to form an aminoacetonitrile product, and
   c) recovering the product,

   wherein
   - the source of hydrogen cyanide in step (a) is the direct unpurified reactor product stream of a hydrogen cyanide production process comprising a gaseous mixture of hydrogen cyanide and ammonia,
   - the source of formaldehyde in step (a) is the direct unpurified reactor product stream of a formaldehyde production process comprising a gaseous mixture of formaldehyde and water.

2. The process according to Claim 1 wherein the process for producing the unpurified hydrogen cyanide reactor product gas stream is selected from the group consisting of the ammoxidation of methane, the ammoxidation of methanol, the reaction of ammonia and propane, the decomposition of formamide, the recovery of hydrogen cyanide from the ammoxidation of propylene, and the process wherein methanol or formaldehyde or a mixture thereof, ammonia and oxygen are reacted in the presence of a metal oxide catalyst simultaneously with the decomposition of formamide.

3. The process according to Claim 1 wherein the process for producing the crude formaldehyde reactor product stream is selected from the group consisting of the catalytic oxidation of methanol over ferric molybdate and the catalytic dehydrogenation of methanol over a silver catalyst.

4. The process according to Claim 1 wherein the reactive absorber is maintained at a pressure in the range 0.35 to 14.1 kg/cm$^2$ (5 to 200 psig.), at a temperature 50° to 90°C., the aqueous scrubber solution has a pH in the range 0.1 to 2.0, the pH being maintained by the continuous addition of a mineral acid, and the nitrogen source is ethylenediamine, to produce ethylene diaminetetraacetonitrile as a product.

5. The process according to Claim 1 wherein (a) the reactive absorber is maintained:
   - at a pressure in the range 0.35 to 14.1 kg/cm$^2$ (5 to 200 psig.) at a temperature in the range 25° to 90°C,
   - the pH of the aqueous scrubber solution is maintained in the range 0.1 to 1.0 by the continuous addition of $H_2SO_4$

   to produce nitrilotriacetonitrile as a product.

6. The process for the production of aminoacetonitriles according to Claim 1 wherein (a) the reactive absorber is maintained:
   - at a pressure in the range 0.07 to 0.70 kg/cm$^2$ (1 to 10 psig.) at a temperature in the range 25° to 85°C,
   - the pH of the aqueous scrubber solution is maintained in the range 8 to 12 by the continuous addition of $NH_3$

   to produce glycinonitrile as a product.

7. The process according to Claim 6 wherein the glycinonitrile product stream is passed through a hot tube reactor maintained at a temperature in the range 60 - 100 ° C.

8. The process according to claim 1 wherein the reactive absorber is maintained:
   - at a pressure in the range 0.35 to 0.70 kg/cm$^2$ (5 to 10 psig) at a temperature in the range ambient to 150 ° C,
   - the pH of the aqueous scrubber solution is maintained in the range 1 to 10 by the continuous addition of acid,

   to product iminodiacetonitrile as a product.

9. The process according to claim 1 wherein the aminoacetonitrile product from step (b) is passed through a hot tube reactor maintained at a temperature in the range 50° to 200° C to accelerate nitrile formation.

**Patentansprüche**

1. Verfahren zum Herstellen von Aminoacetonitrilen durch
   a) Kontaktieren von Cyanwasserstoff, Formaldehyd und einem zusätzlichen Stickstofflieferanten in einem reaktiven Absorber unter reaktiven Bedingungen,
   b) Waschen der Reaktionsprodukte aus (a) oben mit einer pH-kontrollierten, wäßrigen Waschlösung unter Bildung eines Aminoacetonitrilproduktes, und
   c) Isolierung des Produktes, wobei
   - der Cyanwasserstofflieferant in Stufe (a) der unmittelbare ungereinigte Reaktorproduktstrom aus einem Verfahren zum Herstellen von Cyanwasserstoff ist, der ein gasförmiges Gemisch von Cyanwasserstoff und Ammoniak umfaßt,
   - der Formaldehydlieferant in Stufe (a) der unmittelbare ungereinigte Reaktorproduktstrom aus einem Verfahren zum Herstellen von Formaldehyd ist, der ein gasförmiges Gemisch von Formaldehyd und Wasser umfaßt.

2. Verfahren nach Anspruch 1, wobei das Verfahren zum Herstellen des ungereinigten Cyanwasserstoffreaktorproduktgasstroms ausgewählt ist aus der Gruppe bestehend aus der Ammoxidation von Methan, der Ammoxidation von Methanol, der Reaktion von Ammoniak und Propan, der Zersetzung von Amid, der Gewinnung von Cyanwasserstoff aus der Ammoxidation von Propylen, und dem Verfahren, bei dem Methanol oder Formaldehyd oder eine Mischung derselben, Ammoniak und Sauerstoff in Anwesenheit eines Metalloxidkatalysators gleichzeitig mit der Zersetzung von Formamid umgesetzt werden.

3. Verfahren nach Anspruch 1, wobei das Verfahren zum Herstellen des rohen Formaldehydreaktorproduktstroms ausgewählt ist aus der Gruppe bestehend aus der katalytischen Oxidation von Methanol über Eisen-III-Molybdat und der katalytischen Dehydrierung von Methanol über einem Silberkatalysator.

4. Verfahren nach Anspruch 1, wobei der reaktive Absorber bei einem Druck in dem Bereich von 0,35 bis 14,1 kg/cm$^2$ (5 bis 200 psig), bei einer Temperatur von 50 bis 90 ° C gehalten wird, die wäßrige Waschlösung einen pH in dem Bereich von 0,1 bis 2,0 hat, der pH durch kontinuierliche Zugabe einer Mineralsäure aufrechterhalten wird, und der Stickstofflieferant Ethylendiamin ist, um als Produkt Ethylendiamintetraacetonitril herzustellen.

5. Verfahren nach Anspruch 1, wobei (a) der reaktive Absorber
   - bei einem Druck in dem Bereich von 0,35 bis 14,1 kg/cm$^2$ (5 bis 200 psig), bei einer Temperatur in dem Bereich von 25° bis 90 ° C gehalten wird,
   - der pH der wäßrigen Waschlösung durch kontinuierliche Zugabe von $H_2SO_4$ in dem Bereich von 0,1 bis 1,0 gehalten wird, um Nitriloacetonitril als Produkt herzustellen.

6. Verfahren zum Herstellen von Aminoacetonitrilen nach Anspruch 1, wobei (a) der reaktive Absorber
   - bei einem Druck in dem Bereich von 0,07 bis 0,70 kg/cm$^2$ (1 bis 10 psig) bei einer Temperatur in dem Bereich von 25 bis 85 ° C gehalten wird,
   - der pH der wäßrigen Waschlösung in dem Bereich von 8 bis 12 durch kontinuierliche Zugabe von $NH_3$ gehalten wird, um Glycinonitril als Produkt herzustellen.

7. Verfahren nach Anspruch 6, wobei der Glycinonitrilproduktstrom durch einen heißen Rohrreaktor geführt wird, der bei einer Temperatur in dem Bereich von 60 bis 100 °C gehalten wird.

8. Verfahren nach Anspruch 1, wobei der reaktive Absorber
   - bei einem Druck in dem Bereich von 0,35 bis 0,70 kg/cm$^2$ (5 bis 10 psig) bei einer Temperatur in dem Bereich von Zimmertemperatur bis 150 °C gehalten wird,
   - der pH der wäßrigen Waschlösung in dem Bereich von 1 bis 10 durch die kontinuierliche Zugabe von Säure gehalten wird,

   um Iminodiacetonitril als Produkt herzustellen.

9. Verfahren nach Anspruch 1, wobei das Aminoacetonitrilprodukt aus Stufe (b) durch einen heißen Rohrreaktor geführt wird, der bei einer Temperatur in dem Bereich von 50 bis 200 °C gehalten wird, um die Nitrilbildung zu beschleunigen.

## Revendications

1. Procédé pour la production d'aminoacétonitriles préparés par
   a) la mise en contact dans un absorbeur réactif,en conditions réactives de cyanure d'hydrogène, de formaldéhyde et d'une source additionnelle d'azote,
   b) le lavage des produits réactionnels de (a) ci-dessus avec une solution aqueuse de lavage à pH contrôlé pour former un aminoacétonitrile produit, et
   c) la récupération du produit,

   où
   - la source de cyanure d'hydrogène à l'étape (a) est le courant du produit direct et non purifié du réacteur d'un procédé de production de cyanure d'hydrogène comprenant un mélange gazeux de cyanure d'hydrogène et d'ammoniac,
   - la source de formaldéhyde à l'étape (a) est le courant du produit direct et non purifié du réacteur d'un procédé de production de formaldéhyde comprenant un mélange gazeux de formaldéhyde et d'eau.

2. Procédé selon la revendication 1, où le procédé de production du courant gazeux du produit du réacteur de cyanure d'hydrogène non purifié est choisi dans le groupe consistant en ammoxydation du méthane, ammoxydation du méthanol, réaction d'ammoniac et de propane, décomposition du formamide, récupération du cyanure d'hydrogène de l'ammoxydation du propylène et le procédé où on fait réagir le méthanol ou le formaldéhyde ou un mélange, l'ammoniac et l'oxygène en présence d'un oxyde de métal comme catalyseur en même temps que la décomposition du formamide.

3. Procédé selon la revendication 1, où le procédé de production du courant du produit du réacteur de formaldéhyde brut est choisi dans le groupe consistant en oxydation catalytique du méthanol sur du molybdate ferrique et déshydrogénation catalytique du méthanol sur un catalyseur d'argent.

4. Procédé selon la revendication 1, où l'absorbeur réactif est maintenu à une pression comprise entre 0,35 et 14,1 kg/cm$^2$ (5 à 200 psig) à une température de 50 à 90 °C, la solution aqueuse du laveur a un pH compris entre 0,1 et 2,0, le pH étant maintenu par l'addition continue d'un acide minéral et la source d'azote est l'éthylènediamine, pour donner de l'éthylènediaminetétraacétonitrile comme produit.

5. Procédé selon la revendication 1, où (a) l'absorbeur réactif est maintenu :
   - à une pression comprise entre 0,35 et 14,1 kg/cm$^2$ (5 à 200 psig) à une température comprise entre 25 et 90 °C,
   - le pH de la solution du laveur est maintenu entre 0,1 et 1,0 par l'addition continue de $H_2SO_4$

   pour donner du nitrilotriacétonitrile comme produit.

6. Procédé de production d'aminoacétonitriles selon la revendication 1, où (a) l'absorbeur réactif est maintenu :
   - à une pression comprise entre 0,07 et 0,70 kg/cm$^2$ (1 à 10 psig) à une température comprise entre 25 et 85 °C ,
   - le pH de la solution aqueuse de lavage est maintenu entre 8 et 12 par l'addition continue de $NH_3$

   pour donner du glycinonitrile comme produit.

7. Procédé selon la revendication 6, où le courant du glycinonitrile produit passe à travers un réacteur à tube chaud maintenu à une température comprise entre 60 et 100 ° C.

8. Procédé selon la revendication 1, où l'absorbeur réactif est maintenu :
   - à une pression comprise entre 0,35 et 0,70 kg/cm$^2$ (5 à 10 psig) à une température comprise entre l'ambiante et 150 ° C,
   - le pH de la solution aqueuse du laveur est maintenu entre 1 et 10 par addition continue d'un acide

   pour produire de l'iminodiacétonitrile comme produit.

9. Procédé selon la revendication 1, où l'aminoacétonitrile produit à l'étape (b) passe à travers un réacteur à tube chaud maintenu à une température comprise entre 50 et 200 ° C pour accélérer la formation du nitrile.